# EUROPEAN PATENT SPECIFICATION

(11) **EP 4 401 615 B1**
(45) Date of publication and mention of the grant of the patent: **26.11.2025**
(21) Application number: 22777339.7
(22) Date of filing: 05.09.2022
(51) Int. Cl.: A61B 3/10

(54) **DEVICE FOR DIAGNOSING OCULAR SURFACE DISEASES AND KIT FOR THE ADMINISTRATION OF TOPICAL OCULAR THERAPIES BY MEANS OF AEROSOL**
VORRICHTUNG ZUR DIAGNOSE VON AUGENOBERFLÄCHENERKRANKUNGEN UND KIT ZUR VERABREICHUNG TOPISCHER AUGENTHERAPIEN MITTELS AEROSOL
DISPOSITIF PERMETTANT DE DIAGNOSTIQUER DES MALADIES DE SURFACE OCULAIRE ET KIT POUR L'ADMINISTRATION DE THÉRAPIES OCULAIRES TOPIQUES AU MOYEN D'UN AÉROSOL

(30) Priority: 16.09.2021 IT 202100023840
(43) Date of publication of application: 24.07.2024
(73) Proprietor: Frontini, Carlo, 21053 Castellanza (VA) (IT); Compagnone, Emilio, 59290 Wasquehal (FR); Schena, Emiliano, 00128 Roma (RM) (IT); Grasso, Antonella, 83031 Ariano Irpino (AV) (IT); Di Zazzo, Paolo, 86077 Pozzilli (IS) (IT)
(72) Inventor: CRUGLIANO, Salvatore, 20025 Legnano (MI) (IT); DI ZAZZO, Antonio, 00042 Anzio (RM) (IT)
(74) Representative: Barbaro, Gaetano
(86) International application number: PCT/IB2022/058336
(87) International publication number: WO 2023/042030

(56) References cited:
- CN-U- 209 252 827
- US-A- 4 996 993
- US-A1- 2006 157 064
- US-A1- 2007 016 074
- US-A1- 2014 194 706
- US-A1- 2020 359 886

## Description

### Field of the invention

The present invention relates to the definition of the pathological picture and of the treatment of patients suffering from ocular pathologies by proposing an innovative diagnostic tool together with a means for administering the appropriate therapies based on aerosols.

### Prior art

US 2014/194706 discloses contact lens 100 with substrate 102 containing cavity 106 for collecting tear fluid via capillary action/osmosis ([0019]), sensor 108 within cavity for sensing analytes including glucose, alcohol, histamine ([0031]), communication component 610 with RF antenna for wireless transmission ([0050]), processor 640 for determining analyte concentration ([0054]), and external reader device 700 for data processing ([0061]).

US 2007/016074 discloses contact device 2 with rigid annular member 12, flexible membrane 14, movable central piece 16 with flat inner side 24 for corneal applanation ([0582]), facial area positioning against first end 66 of housing 64 ([0618]), and pressure measurement system.

US 4,996,993 discloses in vivo osmometer 10 with probe means 12 having separated electrodes 26, 28 with blunt ends suitable for touching delicate body parts like cornea (column 3, lines 24-28), conductivity measuring means 14, microprocessor 42 for controlling measurements (column 4, line 32), and display means 44 for osmolarity values (column 4, line 57).

US 2020/359886 discloses headset 100 for diagnosing ophthalmic conditions ([0042]) with face mask 805 creating sealed periocular air space 905 covering both eyes ([0054], [0056]), processor 1299 with memory ([0062]), temperature/humidity sensor 1206 with closed-loop control ([0060], [0063]), pupil cameras 422 measuring blink rate, frequency, duration ([0050]), piezoelectric mister 1203, dehumidifier 1207, cooling/heating elements ([0061]), and wireless communication ([0066]).

US 2006/157064 discloses eyewear apparatus with individual curved eyecups 12 having interior cavities 27 for moisture/medicinal treatment, creating "custom fit and effective seal over each eye" and "humidity chamber above and around wearer's eyes" for dry eye therapy, but lacks diagnostic sensors.

CN 209252827U discloses facial paralysis detection device with detection goggles 100 worn on patient's head ([0006]), left/right eye cover cylinders 410, 420 ([0008]), humidity sensors 430, 460 and temperature sensors 440, 470 on inner cylinder walls ([0008]), orbicularis muscle nerve sensors 450, 480 positioned to face patient's eyelid muscles ([0008]), data collection component 200 with display ([0007]), power supply component in installation cavity ([0011]), and flexible rings for comfortable eye area contact ([0035]).

Dry eye disease is a chronic disorder of the ocular surface of multifactorial origin, which severely limits the patient's quality of life. Today it represents one of the most frequent eye diseases in the Western world. The clinical picture manifests itself with ocular discomfort in the initial stages and can extend to corneal damage with risk of ulceration and perforation, and to irreversible scarring of the ocular surface. Dry Eye Disease (DED) is more frequent with advancing age and is a consequence of systemic diseases such as rheumatic, degenerative and hormonal diseases. Such condition can also be caused by chronic local and systemic therapies, while exposure to environmental factors, such as wind, air conditioning, excessive use of contact lenses and video screens are factors that aggravate or trigger the disease.

Currently, dry eye is defined as a "multifactorial disease of the ocular surface characterized by a loss of homeostasis of the tear film, and accompanied by ocular symptoms, in which tear film instability and hyperosmolarity, ocular surface inflammation and damage, and neurosensory abnormalities play etiological roles" (Dews II, 2017).

Therefore it is considered a real disease that affects the complex system of the ocular surface, which is based on the interaction of different tissues such as the lacrimal glands, the eyelids, the cornea and the conjunctiva, but above all the tear film, the local immune system and the neuroendocrine one localized in the mucous membranes. The fundamental feature that gives rise to dry eye is a change in the quantity and composition of the tears, which become denser (lacrimal hyperosmolarity) due to a reduced production of the liquid component by the main lacrimal gland or due to excessive evaporation. The increase in osmolarity in turn causes damage to the epithelial cells of the conjunctiva and the cornea, as well as the goblet cells that produce the normal mucous component of tears, and induces an inflammatory reaction of the entire ocular surface. These alterations trigger a vicious circle that aggravates the dryness situation and causes the process to become chronic.

To date, the diagnosis of dry eye is an "unmet need" of ophthalmology, as all the tests used for the diagnosis are surrogates. The clinical suspicion is confirmed by the evaluation of the ocular surface with the slit lamp and, more particularly, by the finding of a reduced thickness of the lacrimal menisci, that is the layer of tears present between the eyelid rim and the surface of the eyeball. The diagnosis is then clarified by some tests: the evaluation of the break up time (BUT) of the tear film, that is the period of time in which dry areas are formed on the surface of the cornea between one blinking and another; the Schirmer test, which evaluates the length of the soaked portion of a strip of paper inserted in the conjunctival fornix, between the lower eyelid and the eye, over a certain period of time, generally 5 minutes; the tear osmolarity test, which allows a quantitative assessment of the degree of alteration of the tears; and the evaluation of the conditions of the epithelial surface with the help of particular colors (fluorescein, lissamine green, rose bengal).

The object of the present patent is to propose a minimally invasive, rapid, innovative but above all all-encompassing diagnostic method for dry eye disease, based on a device capable of detecting, by means of sensors, the humidity and temperature of the ocular surface, of connecting to a remote device for data processing and possibly of administering an appropriate therapy according to the pathological picture detected. The device which is the subject of the present industrial patent application is therefore a complete system for dry eye diagnosis, prognosis, treatment and follow-up, which can also be used in the diagnosis and treatment of other (infectious and/or inflammatory) diseases of the ocular surface and in the detection of imported biometric data such as glucose concentration. In fact, such device allows not only the qualitative diagnosis of the type of dry eye but also the quantitative one with a single examination, combining diagnostic, prognostic and staging capabilities. As explained below, the two elements considered, namely the diagnostic and the therapeutic, can be used jointly in a single system or structured as two separate entities.

### Description of the invention

According to the present invention, a device for diagnosing ocular surface diseases and a kit for administering the relative therapies by means of aerosols, capable of effectively solving the above problems, are provided. Specifically, the diagnostic device comprises at least an ergonomic terminal with eyecups connected to a data processing base, and can be used both in the diagnosis and in the classification of dry eye syndrome, and in the evaluation of therapeutic progress. The ergonomic terminal with diagnostic functions is designed to be placed in contact with the patient's face and can be indicatively shaped like a helmet, a special eyewear with rear support or a gun held in the operator's hand by means of a handle, having in all cases disposable eyecups or made of sterilizable material which have the purpose of bringing the sensors in the vicinity of the patient's eyeball in a closed and controlled environment.

The eyecups can be of the conventional type or modified for the collection of tear discharge when the humidity reaches 100%, moreover, they can have an absorbent component in contact with the patient's eyelid skin covered with a waterproof layer, to eliminate interference due to sweating. They can be fixed or removable, so that the most suitable sensor is associated with a specific cup.

The sensors placed in the eyepieces of the ergonomic diagnostic terminals can be hygrometer and thermometer, often integrated in a single sensor, suitable to measure relative humidity and temperature, relative humidity (RH) in a given environment is defined as ρω/ρs x100%, where ρω is the water vapor density and ρs is the water vapor density at saturation. Using this sensor, it is possible to collect elements useful for diagnosis and define the difference between those affected by the disease and the normal population.

Other sensors to be applied to the eyecups for the detection of data from the surface of the eye can be: sensors used for the evaluation of the osmolarity/osmolality of the tear discharge; and sensors for contact tear production, sensitive to the progressive imbibition of a support. To all these elements stimuli such as hot, cold or room temperature air, induced change in the humidity rate inside the cup and/or a light stimulus to be stared at can be associated.

It is also possible to insert a micro-camera in the eyecups to evaluate the frequency and dynamics of blinking during the execution of the tests in basal conditions or under stimulus. Furthermore, the system can be implemented with the collection of tear discharge and accurate analysis thereof by means of spectrometry, spectrophotometry or rapid evaluation by colorimetric strips (such as detection of leukocytes, nitrites, PH, glucose concentration, etc.). A further data of diagnostic interest could be that relating to the perception of pain/discomfort felt by the patient both in basal condition and under stimulus. To detect this data and transform it into a digital signal useful for subsequent processing, it could be advantageous to position a pressure sensor at a button mounted on an ergonomic support, this being connected by means of a cable or other wireless system to the processing base or mounted on the processing base itself, through which the patient, when questioned and/or when subjected to a stimulus, expresses his/her assessment of pain/discomfort perceived by pressing the button with higher/lower intensity.

All the data collected by the ergonomic diagnostic terminals, and possibly by the aforementioned optional button for pain assessment, are sent to a data processing base, connected in wired or wireless mode, which stores the data (at any time usable by the operator) and processes the information by applying Artificial Intelligence algorithms such as, by way of non-limiting example, computational statistics, pattern recognition, adaptive filtering, image processing etc., with the aim of providing useful tools to the doctor for a correct diagnosis and treatment of detected pathologies. Such processing base is in turn connected to an external server to which it transmits aggregate data for any further subsequent processing. In a variant, the processing base can be replaced by non-dedicated mobile devices (smartphones, tablets, laptops) or fixed devices (PCs), connected in wired or wireless mode to the diagnostic terminal, suitably provided with hardware capable of supporting adequate dedicated software.

In addition to the diagnostic device, the present invention provides a kit capable of administering any topical ocular therapy normally instillable through eye drops, with the advantage, compared to the latter, of overcoming the missed administration due to blinking, of being able to vary at will the concentrations of the drugs used and of being particularly suitable for uncooperative patients, such as infants or people with disabilities. Such kit comprises a therapeutic terminal implemented by means of a suitably modified aerosol mask, connected to a control base provided with an aerosol device, and a heat source can be associated with it, such as a thermoregulated resistance or an ultrasound emitter, both useful in dry eye syndrome therapy.

Furthermore, the kit can comprise an internal ionizer with the purpose of activating the active ingredients, maximizing the effect thereof and/or facilitating the achievement of the site of action at the level of the entire visual apparatus, allowing to store the drugs in a non-inactive form until the moment of administration, this guarantees the possibility of storing the medicines for a longer time. The above is also valid in the case in which nanoparticles are administered for therapeutic and/or diagnostic purposes.

### Description of the figures

The foregoing advantages, as well as other advantages and features of the present invention, will be illustrated with reference to the accompanying drawings, which are to be considered purely illustrative and not limiting or binding to the effects of the present patent application, in which:

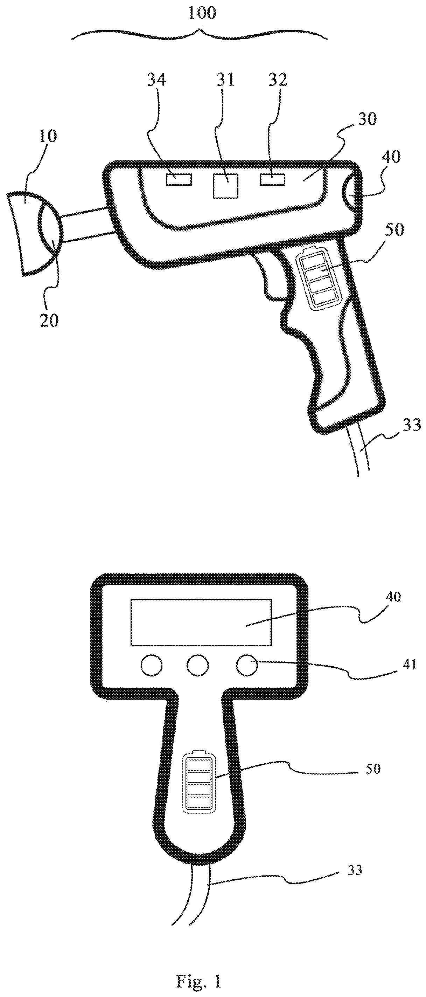

- FIGURE 1 shows a side and rear view of a possible embodiment of a single-sensor "gun" diagnostic terminal 100, where the eyecup 10, the sensor 20 placed inside it, a processing board 30 provided with a processor 31, memory 34 and wireless connection device 32, with the optional connection cable 33, a digital screen 40 with buttons 41 and a rechargeable power supply battery 50 arranged in the handle are visible;

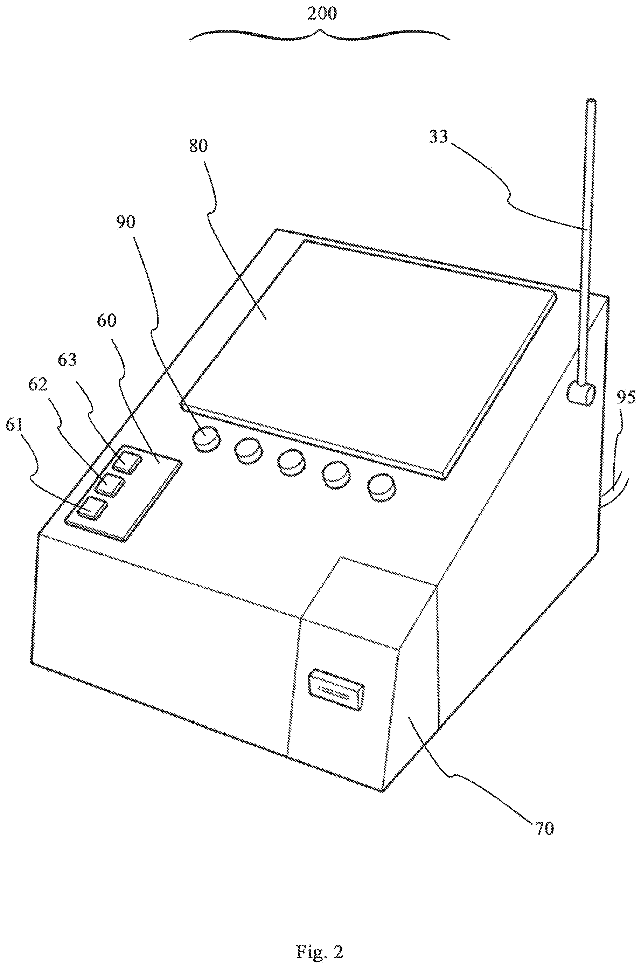

- FIGURE 2 shows a possible embodiment of the processing base 200 where a digital screen 80, menu buttons 90, an optional printer 70, a power supply cable 95, a connection cable 33 which connects to the diagnostic terminal 100, are visible, said base 200 being managed by a processing board 60 having a processor 61, a memory 62 and a remote connection device 63 capable of connecting to the Internet and/or to any remote diagnostic and/or therapeutic terminals;

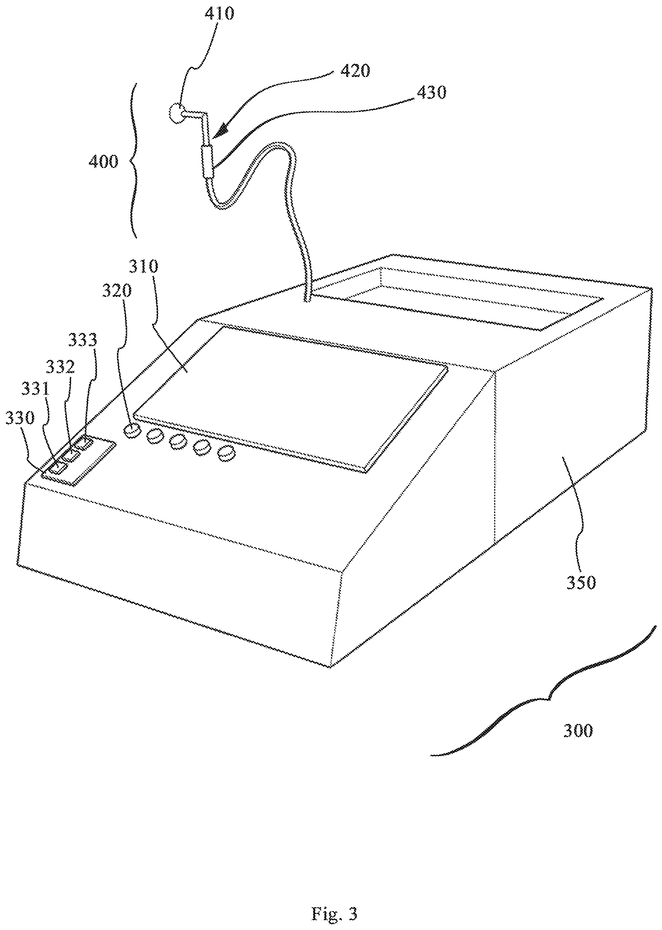

- Figure 3 shows a possible embodiment of the control base 300 for administering the therapy, provided with a computer 330, a display 310, buttons 320, an aerosol device 350 and connected to a therapeutic terminal 400 with an eyepiece dispensing cup 410 and a thermoregulated resistance or other system for varying the temperature of the aerosol 420 in a controlled manner, an ionization system (ionizer) 430;

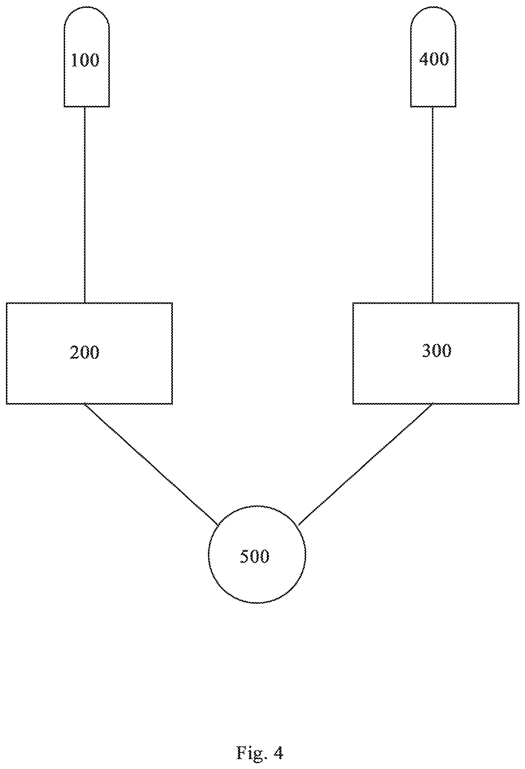

- FIGURE 4 illustrates a possible operating diagram of the device as a whole, where a diagnostic terminal 100 sends the information detected by the ocular surface to a processing base 200 which processes it and sends it to a remote server 500, from which it is downloaded on a control base 300 which is capable of delivering therapy through a therapeutic terminal 400.

### Detailed description of the invention

It will be immediately apparent that innumerable variations and modifications (for example relating to shape, dimensions, arrangements and parts with equivalent functionality) may be made to what has been described without departing from the scope of the invention as appears in the appended claims.

The present invention will now be illustrated, purely by way of non-limiting or binding example, as a diagnostic terminal 100 connected to a processing base 200 by means of a wireless connection, the sensor 20 being installed in the removable eyecup 10 positioned on said terminal 100, a hygrometer/thermometer capable of detecting the relative humidity generated by tear evaporation from the ocular surface. The data detected by the sensor are shown in real time on the digital screen 40 present on the diagnostic terminal 100 for the convenience of the operator, then they are transmitted, by wireless connection, to the processing base 200 which stores and processes them for subsequent integration with previous data, both from the same measurement and from previous measurements, through the application of artificial intelligence algorithms. Said processing base 200 being also able to print them by means of a suitable optional printer 70. The digital data thus integrated and processed are useful for the diagnosis and possible processing of a therapy and are sent to a remote server 500 through an Internet connection, on said server 500 they are also integrated by means of artificial intelligence with a database consisting of data collected from similar devices in use in other locations and are unloaded onto a control base 300 for administering the therapy. Said base 300 displays the prescribed therapy by means of a screen 310 and then delivers the medicine, by means of an integrated aerosol 350, through the therapeutic terminal 400 provided with a suitable eyecup 410, a thermoregulated resistance 420 or other system for varying the temperature of the aerosol, such as an ultrasound emitter, and with an ionization system of the active ingredient, nanoparticle and/or therapeutic substance to be delivered.

Alternatively, the therapeutic data obtained from the processing base 200 can be directly sent to the control base 300. Said data can be viewed by the operator who has complete access and possibility of modification and/or integration at his/her discretion.

It is clear that modifications, additions or variants may be made to the invention described thus far which are apparent to those skilled in the art, without departing from the scope of protection that is provided by the appended claims.

## Claims

1. A device for diagnosing diseases of an ocular surface of a patient, **characterized in that** it comprises:
- at least a processing base (200) provided with a screen (80), push-button panel (90), and an electronic board (60) having a processor (61), a memory (62) and a remote connection device (63), said processing base (200) being suitable to receive and process data coming from a sensor (20) arranged on a diagnostic terminal (100) and said processing base (200) being provided with dedicated software capable of applying, to said data, appropriate artificial intelligence algorithms for providing tools useful to a physician for a correct diagnosis and treatment of detected diseases;
- at least a diagnostic terminal (100) designed to be placed in contact with the patient's face for executing tests to the patient, comprising: at least one fixed or removable eyecup (10) made of disposable or sterilizable material, wherein said eyecup (10) is suitable for inserting a micro-camera for evaluating frequency and dynamics of eye blinking of the patient during execution of said tests to the patient; at least one sensor (20) suitable to detect data from an ocular surface of the patient when in the vicinity of a patient's eyeball; an electronic board (30) having a processor (31), a memory (34) and a remote connection device (32); rechargeable battery (50) for power supply, and a set of buttons (41); said diagnostic terminal (100) is designed in a form chosen among: a single sensor (20) and a single eyecup (10) in the form of a "gun" with handle; or with dual sensor (20) having two distinct eyecups (10) mounted on a helmet for the head of the patient; or with dual sensor (20) having two distinct eyecups (10) mounted on a eyepiece with an extensive eye support for the nape of the patient;
wherein said eyecup (10) is designed to bring the sensor (20) in the vicinity of a patient's eyeball in a closed and controlled environment when the diagnostic terminal (10) is placed in contact with the patient's face, for detecting said data from the ocular surface of the patient.

2. The device for diagnosing ocular surface diseases, according to the preceding claim 1, **characterized in that** the remote connection device (63) present on the processing base (200) is configured to send results of a processing carried out on the data obtained from the diagnostic terminal (100) to a remote server (500), said data being processable subsequently, i.e. shared, or downloaded from a control base (300), suitably identified by means of a unique alphanumeric code, for a subsequent administration of a therapy.

3. The device for diagnosing ocular surface diseases according to any one of the preceding claims, **characterized in that** the processing base (200) consists of a non-dedicated external mobile or fixed device, such as a fixed or portable computer, a mobile phone, a tablet or other remote device, suitably provided with hardware capable of supporting a dedicated software and connected to the diagnostic terminal (100) in wired or wireless mode.

4. The device for diagnosing ocular surface diseases according to any one of the preceding claims, **characterized in that** the diagnostic terminal (100) is provided with a digital screen (40) for an immediate display of a datum of said data detected by the sensor (20), in order to allow an operator a better understanding of the detected datum.

5. The device for diagnosing ocular surface diseases according to claim 4, **characterized in that** said digital screen (80) present on the processing base (200) allows an operator to continuously display diagnostic data, said continuous display of data also being allowed on the digital screen (40) of the diagnostic terminal (100).

6. The device for diagnosing ocular surface diseases, according to any one of the preceding claims, **characterized in that** said eyecup (10) is suitably modified for the collection of tear discharge when a relative humidity detected by the sensor (20) reaches 100%; said processing base (200) comprising a spectrometer or spectrophotometer for examining said tear discharge by means of said artificial intelligence algorithms.

7. The device for diagnosing ocular surface diseases, according to any one of the preceding claims, **characterized in that** the sensor (20) positioned inside the eyecup (10) of the diagnostic terminal (100) is a sensor used to evaluate osmolarity/osmolality of a tear discharge.

8. The device for diagnosing ocular surface diseases, according to any one of the preceding claims, **characterized in that** the sensor (20) positioned inside the eyecup (10) of the diagnostic terminal (100) is a hygrometer/thermometer sensor suitable to detect relative humidity and temperature in the closed environment created around the ocular surface by the eyecup (10).

9. The device for diagnosing ocular surface diseases, according to any one of the preceding claims, **characterized in that** the processing base (200) is connected to a button provided with a pressure sensor, suitable to detect pain/discomfort felt by the patient in basal condition and/or under stimulus, suitable to detect data detected by said button and to send them to the electronic board (60) positioned on said processing base (200) for subsequent integration and processing; said button being mounted directly on the processing base (200) or on a handle or other ergonomic support to be handed to the patient and suitably connected to the processing base (200).

10. The device for diagnosing ocular surface diseases, according to any one of the preceding claims, **characterized in that** the processing base (200) is provided with a printer (70) suitable for printing medical receipts containing the detected data, assigned therapies and/or other information useful for diagnosis and therapy according to a judgment of the physician.

11. The device for diagnosing ocular surface diseases, according to any one of the preceding claims, **characterized in that** the eyecup (10) mounted on the diagnostic terminal (100) is provided with a micro-camera, positioned next to the sensor (20) or in another position inside said eyecup (10), suitable to evaluate the frequency and dynamics of the blinking and closing of a patient's eyelid when running the tests in basal conditions and under stimulus, said micro-camera being configured to shown captured videos on the digital screen (80) present on said processing base (200).

12. The device for diagnosing ocular surface diseases, according to any one of the preceding claims, **characterized in that** the eyecup (10) mounted on the diagnostic terminal (100) is provided with a device, positioned next to the sensor (20) or in another position inside said eyecup (10) suitable to dispense air blows at various temperatures and suitable to run diagnostic tests on the ocular surface.

13. The device for diagnosing ocular surface diseases, according to any one of the preceding claims, **characterized in that** the eyecup (10) mounted on the diagnostic terminal (100) is provided with a device, positioned next to the sensor (20), suitable to emit a luminous stimulus at various intensities and suitable to run diagnostic tests on the ocular surface.

14. The device for diagnosing ocular surface diseases, according to any one of the preceding claims, **characterized in that** the eyecup (10) mounted on the diagnostic terminal (100) is provided with a device, positioned next to the sensor (20), suitable to induce a variation in the humidity level of the closed environment inside said eyecup (10), for running diagnostic tests on an ocular surface of the patient.

15. The device for diagnosing ocular surface diseases, according to any one of the preceding claims, **characterized in that** said diagnostic terminal (100) and said processing base (200) are connected by a cable (33) suitable to transmit data and/or recharge the battery (50) positioned on the handle of said computer (100).

16. A kit for the administration of topical ocular therapies by means of aerosol, **characterized in that** it comprises:
- the device for diagnosing according to one of the preceding claims;
- at least a control base (300) provided with a screen (310), push-button panel (320), aerosol device (350) and electronic board (330) having a processor (331), memory (332) and remote connection device (333), said control base (300) being suitable to administer any topical therapy avoiding non-administration due to blinking, being suitable to vary concentrations of drugs used at will and being particularly suitable for non-collaborative patients, such as new-borns or persons with disabilities;
- at least a therapeutic computer (400), suitable to deliver a prescribed medicinal product as therapy following a diagnosis carried out with the device for diagnosing of any one of the preceding claims or by direct entry of therapeutic data by the physician in the control base (300), connected to said control base (300) and comprising an eyecup (410) to be brought into contact with the face of the patient and a thermoregulated resistor (420) or other aerosol thermoregulation system.

17. The kit for the administration of topical ocular therapies by means of aerosol, according to claim 16, **characterized in that** the therapeutic computer (400) connected to the control base (300) is provided with a system (430) for ionizing the drugs, nanoparticle and/or therapeutic substances to be dispensed, and for storing the drugs in a non-active form until the moment of administration.

18. The kit according to claim 16 or 17, **characterized in that** the processing base (200) and the control base (300) are integrated in a single diagnostic/therapeutic device.

## Patentansprüche

1. Vorrichtung zum Diagnostizieren von Erkrankungen einer Augenoberfläche eines Patienten, **dadurch gekennzeichnet, dass** es umfasst:
- mindestens eine Verarbeitungsbasis (200), die mit einem Bildschirm (80), einem Tastenfeld (90) und einer elektronischen Platine (60) versehen ist, aufweisend einen Prozessor (61), einen Speicher (62) und eine Fernverbindungsvorrichtung (63), wobei die Verarbeitungsbasis (200) geeignet ist, um Daten zu empfangen und zu verarbeiten, die von einem Sensor (20) stammen, der auf einem Diagnoseendgerät (100) angeordnet ist, und wobei die Verarbeitungsbasis (200) mit einer speziellen Software versehen ist, die in der Lage ist, auf die Daten geeignete Algorithmen künstlicher Intelligenz anzuwenden, zum Bereitstellen von nützlichen Werkzeugen für eine korrekte Diagnose und Behandlung erfasster Krankheiten an einen Arzt;
- mindestens ein Diagnoseendgerät (100), das bestimmt ist, um in Kontakt mit dem Gesicht des Patienten gebracht zu werden, zum Ausführen von Tests an dem Patienten, umfassend: mindestens eine feste oder abnehmbare Augenmuschel (10), die aus Einweg- oder sterilisierbarem Material hergestellt ist, wobei die Augenmuschel (10) zum Einsetzen einer Mikrokamera geeignet ist, zum Bewerten einer Frequenz und Dynamik eines Augenblinzelns des Patienten während der Ausführung der Tests an dem Patienten; mindestens einen Sensor (20), der geeignet ist, Daten von einer Augenoberfläche des Patienten zu erfassen, wenn in der Nähe eines Augapfels des Patienten; eine elektronische Platine (30), die einen Prozessor (31), einen Speicher (34) und einen Fernverbindungsvorrichtung (32) aufweist; wiederaufladbare Batterie (50) für eine Leistungsversorgung und einen Satz von Knöpfen (41); wobei das Diagnoseendgerät (100) in einer Form gestaltet ist, die ausgewählt ist aus: einem einzelnen Sensor (20) und einer einzelnen Augenmuschel (10) in der Form einer "Pistole" mit Griff; oder mit einem Doppelsensor (20), der zwei unterschiedliche Augenmuscheln (10) aufweist, die auf einem Helm für den Kopf des Patienten montiert sind; oder mit einem Doppelsensor (20), der zwei unterschiedliche Augenmuscheln (10) aufweist, die auf einem Okular mit einer umfangreichen Augenstütze für den Nacken des Patienten montiert sind;
wobei die Augenmuschel (10) gestaltet ist, um den Sensor (20) in einer geschlossenen und gesteuerten Umgebung in die Nähe des Augapfels eines Patienten zu bringen, wenn das Diagnoseendgerät (10) in Kontakt mit dem Gesicht des Patienten gebracht wird, zum Erfassen der Daten von der Augenoberfläche des Patienten.

2. Vorrichtung zum Diagnostizieren von Erkrankungen der Augenoberfläche nach dem vorstehenden Anspruch 1, **dadurch gekennzeichnet, dass** die auf der Verarbeitungsbasis (200) vorhandene Fernverbindungsvorrichtung (63) konfiguriert ist, um Ergebnisse einer Verarbeitung, die an den Daten ausgeführt werden, die von dem Diagnoseendgerät (100) erhalten werden, an einen entfernten Server (500) zu senden, wobei die Daten anschließend verarbeitet, d. h. geteilt, oder von einer Steuerbasis (300), die mittels eines eindeutigen alphanumerischen Codes auf geeignete Weise identifiziert wird, für eine anschließende Verabreichung einer Therapie heruntergeladen werden können.

3. Vorrichtung zum Diagnostizieren von Erkrankungen der Augenoberfläche nach einem der vorstehenden Ansprüche, **dadurch gekennzeichnet, dass** die Verarbeitungsbasis (200) aus einer nicht dedizierten externen mobilen oder festen Vorrichtung, wie etwa einem festen oder tragbaren Computer, einem Mobiltelefon, einem Tablet oder einer anderen entfernten Vorrichtung besteht, die auf geeignete Weise mit Hardware versehen ist, die in der Lage ist, eine dedizierte Software zu unterstützen, und in dem drahtgebundenen oder drahtlosen Modus mit dem Diagnoseendgerät (100) verbunden zu sein.

4. Vorrichtung zum Diagnostizieren von Erkrankungen der Augenoberfläche nach einem der vorstehenden Ansprüche, **dadurch gekennzeichnet, dass** das Diagnoseendgerät (100) mit einem digitalen Bildschirm (40) für eine sofortigen Anzeige eines Datums der durch den Sensor (20) erfassten Daten versehen ist, um einem Bediener ein besseres Verständnis des erfassten Datums zu ermöglichen.

5. Vorrichtung zum Diagnostizieren von Erkrankungen der Augenoberfläche nach Anspruch 4, **dadurch gekennzeichnet, dass** der digitale Bildschirm (80), der auf der Verarbeitungsbasis (200) vorhanden ist, einem Bediener ermöglicht, kontinuierlich Diagnosedaten anzuzeigen, wobei die kontinuierliche Anzeige von Daten ebenso auf dem digitalen Bildschirm (40) des Diagnoseendgeräts (100) möglich ist.

6. Vorrichtung zum Diagnostizieren von Erkrankungen der Augenoberfläche nach einem der vorstehenden Ansprüche, **dadurch gekennzeichnet, dass** die Augenmuschel (10) auf geeignete Weise modifiziert ist, um Tränenausfluss aufzufangen, wenn eine durch den Sensor (20) erfasste relative Luftfeuchtigkeit 100 % erreicht; die Verarbeitungsbasis (200) umfassend ein Spektrometer oder Spektrophotometer zum Untersuchen des Tränenausflusses mittels der Algorithmen der künstlichen Intelligenz.

7. Vorrichtung zum Diagnostizieren von Erkrankungen der Augenoberfläche nach einem der vorstehenden Ansprüche, **dadurch gekennzeichnet, dass** der Sensor (20), der in der Augenmuschel (10) des Diagnoseendgeräts (100) positioniert ist, ein Sensor ist, der verwendet wird, um Osmolarität/Osmolalität eines Tränenausflusses zu bewerten.

8. Vorrichtung zum Diagnostizieren von Erkrankungen der Augenoberfläche nach einem der vorstehenden Ansprüche, **dadurch gekennzeichnet, dass** der Sensor (20), der in der Augenmuschel (10) des Diagnoseendgeräts (100) positioniert ist, ein Hygrometer-/Thermometersensor ist, der geeignet ist, um die relative Luftfeuchtigkeit und Temperatur in der geschlossenen Umgebung zu messen, die durch die Augenmuschel (10) um die Augenoberfläche herum geschaffen wird.

9. Vorrichtung zum Diagnostizieren von Erkrankungen der Augenoberfläche nach einem der vorstehenden Ansprüche, **dadurch gekennzeichnet, dass** die Verarbeitungsbasis (200) mit einem Knopf verbunden ist, der mit einem Drucksensor versehen ist, der geeignet ist, Schmerz/Unwohlsein zu erfassen, den/das der Patient in einem Grundzustand und/oder unter Reizung empfindet, und der geeignet ist, um Daten, die durch den Knopf erfasst werden, zu erfassen und sie für anschließendes Integrieren und Verarbeiten an die elektronische Platine (60) zu senden, die auf der Verarbeitungsbasis (200) positioniert ist; wobei der Knopf direkt auf der Verarbeitungsbasis (200) oder auf einem Griff oder einer anderen ergonomischen Stütze montiert ist, die dem Patienten gereicht wird und auf geeignete Weise mit der Verarbeitungsbasis (200) verbunden ist.

10. Vorrichtung zum Diagnostizieren von Erkrankungen der Augenoberfläche nach einem der vorstehenden Ansprüche, **dadurch gekennzeichnet, dass** die Verarbeitungsbasis (200) mit einem Drucker (70) versehen ist, der zum Ausdrucken medizinischer Quittungen geeignet ist, die die erfassten Daten, zugewiesene Therapien und/oder andere für die Diagnose und Therapie nach Einschätzung des Arztes nützliche Informationen enthalten.

11. Vorrichtung zum Diagnostizieren von Erkrankungen der Augenoberfläche nach einem der vorstehenden Ansprüche, **dadurch gekennzeichnet, dass** die Augenmuschel (10), die auf dem Diagnoseendgerät (100) montiert ist, mit einer Mikrokamera versehen ist, die neben dem Sensor (20) oder an einer anderen Position innerhalb der Augenmuschel (10) positioniert ist, die geeignet ist, um die Frequenz und Dynamik des Blinzelns und Schließens des Augenlids eines Patienten zu bewerten, wenn die Tests in einem Grundzustand und unter Reizung durchgeführt werden, wobei die Mikrokamera konfiguriert ist, um aufgenommene Videos auf dem digitalen Bildschirm (80) zu zeigen, der auf der Verarbeitungsbasis (200) vorhanden ist.

12. Vorrichtung zum Diagnostizieren von Erkrankungen der Augenoberfläche nach einem der vorstehenden Ansprüche, **dadurch gekennzeichnet, dass** die Augenmuschel (10), die auf dem Diagnoseendgerät (100) montiert ist, mit einer Vorrichtung versehen ist, die neben dem Sensor (20) oder an einer anderen Position innerhalb der Augenmuschel (10) positioniert ist, die geeignet ist, um Luftstöße mit unterschiedlichen Temperaturen abzugeben und Diagnosetests an der Augenoberfläche vorzunehmen.

13. Vorrichtung zum Diagnostizieren von Erkrankungen der Augenoberfläche nach einem der vorstehenden Ansprüche, **dadurch gekennzeichnet, dass** die Augenmuschel (10), die auf dem Diagnoseendgerät (100) montiert ist, mit einer Vorrichtung versehen ist, die neben dem Sensor (20) positioniert ist, die geeignet ist, um einen Lichtreiz mit unterschiedlicher Intensität auszusenden und Diagnosetests an der Augenoberfläche vorzunehmen.

14. Vorrichtung zum Diagnostizieren von Erkrankungen der Augenoberfläche nach einem der vorstehenden Ansprüche, **dadurch gekennzeichnet, dass** die Augenmuschel (10), die auf dem Diagnoseendgerät (100) montiert ist, mit einer Vorrichtung versehen ist, die neben dem Sensor (20) positioniert ist, die geeignet ist, um eine Veränderung des Feuchtigkeitsniveaus der geschlossenen Umgebung innerhalb der Augenmuschel (10) herbeizuführen, um Diagnosetests an einer Augenoberfläche des Patienten vorzunehmen.

15. Vorrichtung zum Diagnostizieren von Erkrankungen der Augenoberfläche nach einem der vorstehenden Ansprüche, **dadurch gekennzeichnet, dass** das Diagnoseendgerät (100) und die Verarbeitungsbasis (200) durch ein Kabel (33) verbunden sind, das geeignet ist, um Daten zu übertragen und/oder die Batterie (50) wieder aufzuladen, die an dem Griff des Computers (100) positioniert ist.

16. Kit für die Verabreichung topischer Augentherapien mittels Aerosol, **dadurch gekennzeichnet, dass** es umfasst:
- die Vorrichtung zum Diagnostizieren nach einem der vorstehenden Ansprüche;
- mindestens eine Steuerbasis (300), die mit einem Bildschirm (310), einem Tastenfeld (320), einer Aerosolvorrichtung (350) und einer elektronischen Platine (330) versehen ist, aufweisend einen Prozessor (331), einen Speicher (332) und eine Fernverbindungsvorrichtung (333), wobei die Steuerbasis (300) geeignet ist, um beliebige topische Therapien zu verabreichen und eine Nichtverabreichung aufgrund von Blinzeln zu vermeiden, geeignet ist, um Konzentrationen der verwendeten Medikamente beliebig zu variieren und insbesondere für nicht kooperative Patienten wie Neugeborene oder Menschen mit Behinderungen geeignet ist;
- mindestens einen Therapiecomputer (400), der geeignet ist, um ein verschriebenes Arzneimittel als Therapie im Anschluss an eine mit der Diagnosevorrichtung nach einem der vorstehenden Ansprüche durchgeführte Diagnose oder durch direkte Eingabe therapeutischer Daten durch den Arzt in die Steuerbasis (300) abzugeben, der mit der Steuerbasis (300) verbunden ist und umfassend eine Augenmuschel (410), die mit dem Gesicht des Patienten in Kontakt gebracht wird, und einen thermoregulierten Widerstand (420) oder ein anderes Aerosol-Thermoregulierungssystem.

17. Kit für die Verabreichung topischer Augentherapien mittels Aerosol nach Anspruch 16, **dadurch gekennzeichnet, dass** der Therapiecomputer (400), der mit der Steuerbasis (300) verbunden ist, mit einem System (430) zum Ionisieren der abzugebenden Medikamente, Nanopartikel und/oder therapeutischen Substanzen und zum Speichern der Medikamente in nicht aktiver Form bis zu einem Zeitpunkt der Verabreichung versehen ist.

18. Kit nach Anspruch 16 oder 17, **dadurch gekennzeichnet, dass** die Verarbeitungsbasis (200) und die Steuerbasis (300) in einer einzigen Diagnose- /Therapievorrichtung integriert sind.

## Revendications

1. Dispositif de diagnostic de maladies d'une surface oculaire d'un patient, **caractérisé en ce qu'il** comprend :
- au moins une base de traitement (200) dotée d'un écran (80), d'un panneau à boutons-poussoirs (90), et d'une carte électronique (60) ayant un processeur (61), une mémoire (62) et un dispositif de connexion à distance (63), ladite base de traitement (200) étant apte à recevoir et à traiter des données provenant d'un capteur (20) agencé sur un terminal de diagnostic (100) et ladite base de traitement (200) étant dotée d'un logiciel dédié capable d'appliquer, auxdites données, des algorithmes d'intelligence artificielle appropriés permettant de fournir des outils utiles à un médecin en vue d'un diagnostic et d'un traitement corrects de maladies détectées ;
- au moins un terminal de diagnostic (100) conçu pour être placé en contact avec le visage du patient afin d'effectuer des tests sur le patient, comprenant : au moins un œilleton (10) fixe ou amovible en matériau jetable ou stérilisable, dans lequel ledit œilleton (10) est apte à l'insertion d'une micro-caméra permettant d'évaluer la fréquence et la dynamique du clignement des yeux du patient pendant l'exécution desdits tests sur le patient ; au moins un capteur (20) apte à détecter des données provenant d'une surface oculaire du patient lorsqu'il se trouve à proximité d'un globe oculaire du patient ; une carte électronique (30) ayant un processeur (31), une mémoire (34) et un dispositif de connexion à distance (32) ; une batterie rechargeable (50) pour l'alimentation électrique, et un ensemble de boutons (41) ; ledit terminal de diagnostic (100) est conçu sous une forme choisie parmi : un seul capteur (20) et un seul œilleton (10) en forme de « pistolet » avec poignée ; ou avec un double capteur (20) ayant deux œilletons (10) distincts, montés sur un casque pour la tête du patient ; ou avec un double capteur (20) ayant deux œilletons (10) distincts, montés sur un oculaire avec un support oculaire étendu pour la nuque du patient ;
dans lequel ledit œilleton (10) est conçu pour amener le capteur (20) à proximité d'un globe oculaire du patient dans un environnement fermé et régulé lorsque le terminal de diagnostic (10) est placé en contact avec le visage du patient, afin de détecter lesdites données provenant de la surface oculaire du patient.

2. Dispositif de diagnostic de maladies de surface oculaire, selon la revendication 1 précédente, **caractérisé en ce que** le dispositif de connexion à distance (63) présent sur la base de traitement (200) est configuré pour envoyer des résultats d'un traitement effectué sur les données obtenues du terminal de diagnostic (100) à un serveur à distance (500), lesdites données pouvant être traitées ultérieurement, c'est-à-dire partagées, ou téléchargées à partir d'une base de commande (300), convenablement identifiée au moyen d'un code alphanumérique unique, pour une administration ultérieure d'une thérapie.

3. Dispositif de diagnostic de maladies de surface oculaire selon l'une quelconque des revendications précédentes, **caractérisé en ce que** la base de traitement (200) consiste en un dispositif externe mobile ou fixe, non dédié, tel qu'un ordinateur fixe ou portable, un téléphone mobile, une tablette ou un autre dispositif distant, convenablement équipé d'un matériel capable de prendre en charge un logiciel dédié et connecté au terminal de diagnostic (100) en mode filaire ou sans fil.

4. Dispositif de diagnostic de maladies de surface oculaire selon l'une quelconque des revendications précédentes, **caractérisé en ce que** le terminal de diagnostic (100) est doté d'un écran numérique (40) pour un affichage immédiat d'une donnée desdites données détectées par le capteur (20), afin de permettre à un opérateur une meilleure compréhension de la donnée détectée.

5. Dispositif de diagnostic de maladies de surface oculaire selon la revendication 4, **caractérisé en ce que** ledit écran numérique (80) présent sur la base de traitement (200) permet à un opérateur d'afficher en continu des données de diagnostic, ledit affichage continu de données étant également permis sur l'écran numérique (40) du terminal de diagnostic (100).

6. Dispositif de diagnostic de maladies de surface oculaire, selon l'une quelconque des revendications précédentes, **caractérisé en ce que** ledit œilleton (10) est modifié de manière appropriée pour la collecte d'un écoulement lacrymal lorsqu'une humidité relative détectée par le capteur (20) atteint 100 % ; ladite base de traitement (200) comprenant un spectromètre ou un spectrophotomètre permettant d'examiner ladite décharge lacrymale au moyen desdits algorithmes d'intelligence artificielle.

7. Dispositif de diagnostic de maladies de surface oculaire, selon l'une quelconque des revendications précédentes, **caractérisé en ce que** le capteur (20) positionné à l'intérieur de l'œilleton (10) du terminal de diagnostic (100) est un capteur utilisé pour évaluer l'osmolarité/osmolalité d'un écoulement lacrymal.

8. Dispositif de diagnostic de maladies de surface oculaire, selon l'une quelconque des revendications précédentes, **caractérisé en ce que** le capteur (20) positionné à l'intérieur de l'œilleton (10) du terminal de diagnostic (100) est un capteur d'hygromètre/thermomètre apte à détecter l'humidité relative et la température dans l'environnement fermé créé autour de la surface oculaire par l'œilleton (10).

9. Dispositif de diagnostic de maladies de surface oculaire, selon l'une quelconque des revendications précédentes, **caractérisé en ce que** la base de traitement (200) est connectée à un bouton doté d'un capteur de pression, apte à détecter une douleur/gêne ressentie par le patient en condition basale et/ou sous stimulus, apte à détecter des données détectées par ledit bouton et à les envoyer à la carte électronique (60) positionnée sur ladite base de traitement (200) pour une intégration et un traitement ultérieurs ; ledit bouton étant monté directement sur la base de traitement (200) ou sur une poignée ou un autre support ergonomique à remettre au patient et connecté de manière appropriée à la base de traitement (200).

10. Dispositif de diagnostic de maladies de surface oculaire, selon l'une quelconque des revendications précédentes, **caractérisé en ce que** la base de traitement (200) est dotée d'une imprimante (70) apte à imprimer des reçus médicaux contenant les données détectées, des thérapies attribuées et/ou d'autres informations utiles pour le diagnostic et la thérapie selon le jugement du médecin.

11. Dispositif de diagnostic de maladies de surface oculaire, selon l'une quelconque des revendications précédentes, **caractérisé en ce que** l'œilleton (10) monté sur le terminal de diagnostic (100) est doté d'une micro-caméra, positionnée à côté du capteur (20) ou dans une autre position à l'intérieur dudit œilleton (10), apte à évaluer la fréquence et la dynamique du clignement et de la fermeture d'une paupière du patient lors de la réalisation des tests dans des conditions basales et sous stimulus, ladite micro-caméra étant configurée pour montrer des vidéos capturées sur l'écran numérique (80) présent sur ladite base de traitement (200).

12. Dispositif de diagnostic de maladies de surface oculaire, selon l'une quelconque des revendications précédentes, **caractérisé en ce que** l'œilleton (10) monté sur le terminal de diagnostic (100) est doté d'un dispositif, positionné à côté du capteur (20) ou dans une autre position à l'intérieur dudit œilleton (10), apte à distribuer des souffles d'air à différentes températures et apte à effectuer des tests de diagnostic sur la surface oculaire.

13. Dispositif de diagnostic de maladies de surface oculaire, selon l'une quelconque des revendications précédentes, **caractérisé en ce que** l'œilleton (10) monté sur le terminal de diagnostic (100) est doté d'un dispositif, positionné à côté du capteur (20), apte à émettre un stimulus lumineux à différentes intensités et apte à effectuer des tests de diagnostic sur la surface oculaire.

14. Dispositif de diagnostic de maladies de surface oculaire, selon l'une quelconque des revendications précédentes, **caractérisé en ce que** l'œilleton (10) monté sur le terminal de diagnostic (100) est doté d'un dispositif, positionné à côté du capteur (20), apte à induire une variation du taux d'humidité de l'environnement fermé à l'intérieur dudit œilleton (10), pour la réalisation de tests de diagnostic sur une surface oculaire du patient.

15. Dispositif de diagnostic de maladies de surface oculaire, selon l'une quelconque des revendications précédentes, **caractérisé en ce que** ledit terminal de diagnostic (100) et ladite base de traitement (200) sont connectés par un câble (33) apte à transmettre des données et/ou à recharger la batterie (50) positionnée sur la poignée dudit ordinateur (100).

16. Kit pour l'administration de thérapies oculaires topiques au moyen d'un aérosol, **caractérisé en ce qu'il** comprend :
- le dispositif de diagnostic selon l'une des revendications précédentes ;
- au moins une base de commande (300) dotée d'un écran (310), d'un panneau à boutons-poussoirs (320), d'un dispositif d'aérosol (350) et d'une carte électronique (330) ayant un processeur (331), une mémoire (332) et un dispositif de connexion à distance (333), ladite base de commande (300) étant appropriée pour administrer toute thérapie topique en évitant une non-administration due au clignement des yeux, étant apte à faire varier des concentrations de médicaments utilisés à volonté et convenant particulièrement à des patients non coopératifs, tels que des nouveau-nés ou des personnes en situation de handicap ;
- au moins un ordinateur thérapeutique (400), apte à délivrer un produit médicinal prescrit comme thérapie suite à un diagnostic effectué avec le dispositif de diagnostic selon l'une quelconque des revendications précédentes ou par saisie directe de données thérapeutiques par le médecin dans la base de commande (300), connecté à ladite base de commande (300) et comprenant un œilleton (410) à mettre en contact avec le visage du patient et une résistance thermorégulée (420) ou un autre système de thermorégulation d'aérosol.

17. Kit pour l'administration de thérapies oculaires topiques au moyen d'un aérosol, selon la revendication 16, **caractérisé en ce que** l'ordinateur thérapeutique (400) connecté à la base de commande (300) est doté d'un système (430) permettant d'ioniser les médicaments, les nanoparticules et/ou les substances thérapeutiques à distribuer, et de stocker les médicaments sous une forme non active jusqu'au moment de l'administration.

18. Kit selon la revendication 16 ou 17, **caractérisé en ce que** la base de traitement (200) et la base de commande (300) sont intégrées dans un seul dispositif de diagnostic/thérapeutique.
